# EUROPEAN PATENT APPLICATION

(11) **EP 4 737 562 A1**
(43) Date of publication of application: **06.05.2026**
(21) Application number: 24832087.1
(22) Date of filing: 28.06.2024
(51) Int. Cl.: C12N 5/079, C12N 5/0793, C12N 5/10, C12Q 1/02

(54) **METHOD FOR PRODUCING THREE-DIMENSIONAL CELL CULTURE, METHOD FOR CULTURING THREE-DIMENSIONAL CELL CULTURE, THREE-DIMENSIONAL CELL CULTURE, AND METHOD FOR EVALUATING TEST SUBSTANCE**

(30) Priority: 30.06.2023 JP 2023108777
(71) Applicant: FUJIFILM Corporation, Tokyo 106-8620 (JP)
(72) Inventor: KOBAYASHI Hayato, Ashigarakami-gun, Kanagawa 258-8577 (JP); ENDOH Setsu, Ashigarakami-gun, Kanagawa 258-8577 (JP); KATO Hiroshi, Ashigarakami-gun, Kanagawa 258-8577 (JP)
(74) Representative: Hoffmann Eitle
(86) International application number: PCT/JP2024/023479
(87) International publication number: WO 2025/005229

(57) **Abstract**

An object of the present invention is to provide a production method and a culture method of a three-dimensional cell culture containing human-derived neurons, human-derived astrocytes, and human-derived microglia capable of mimicking human brain functions; a three-dimensional cell culture containing human-derived neurons, human-derived astrocytes, and human-derived microglia capable of mimicking human brain functions; and an evaluation method of a test substance using the three-dimensional cell culture. According to the present invention, there is provided a production method of a three-dimensional cell culture, the production method including a step of adding, prior to co-culture of cells, human-derived astrocytes, human-derived neurons, and human-derived microglia to a culture vessel, and a step of co-culturing the astrocytes, the neurons, and the microglia in the culture vessel, in which a proportion of the astrocytes among all cells added to the culture vessel is 30% or more.

## Description

### Technical Field

The present invention relates to a production method of a three-dimensional cell culture and a culture method of a three-dimensional cell culture, using astrocytes, neurons, and microglia. Furthermore, the present invention relates to a three-dimensional cell culture containing astrocytes, neurons, and microglia. Moreover, the present invention relates to an evaluation method of a test substance using the three-dimensional cell culture.

### Background Art

The central nervous system is composed of neurons and glial cells (astrocytes, microglia, and oligodendrocytes), and these cells exhibit normal brain functions by influencing each other (Non-Patent Document 1). For example, the neurons transmit information to other neurons by releasing glutamic acid into a synaptic cleft, and the astrocytes play a role in regulating the information transmission of the neurons by taking up glutamic acid (Non-Patent Document 2). In addition, it has been reported that microglia are activated by aging or inflammation to affect astrocytes, and the astrocytes further damage neurons (Non-Patent Documents 3 and 4). Therefore, a co-culture system capable of evaluating the intercellular interactions between neurons and glial cells is very useful for research on the central nervous system.

In addition, in recent years, various three-dimensional culture systems have been reported to mimic the complicated structure of the human brain (Non-Patent Document 5). Furthermore, it is also known that there are species differences in neurons and glial cells (Non-Patent Documents 6 and 7), and it is very important to use human cells to reproduce the human brain.

Patent Document 1 describes a three-dimensional co-culture containing astrocytes, neurons, and microglia derived from human iPS cells. Patent Document 2 describes a three-dimensional organoid containing neurons and glia derived from human iPS cells. Patent Document 3 describes a three-dimensional (spheroid) co-culture containing neurons and glia derived from human iPS cells.

### Prior Art Documents

### Patent Documents

Patent Document 1: WO2023/039567A
Patent Document 2: WO2022/183015A
Patent Document 3: WO2022/178395A

### Non-Patent Documents

Non-Patent Document 1: Francesca Cerbai et al., PLoS One, Vol. 7, Article number e45250, 2012
Non-Patent Document 2: Jens V. Andersen et al., Neuropharmacology, Vol. 14, Article No. 108719, 2021
Non-Patent Document 3: Shane A. Liddelow et al., Nature, Vol. 541, pp. 481-487, 2017
Non-Patent Document 4: Laura E. Clarke et al., Proceedings of the National Academy of Sciences of the United States of America, Vol. 115, pp. E1896-E1905, 2018
Non-Patent Document 5: Eduarda G. Z. Centeno et al., Molecular Neurodegeneration, Vol. 13, Article No. 27, 2018
Non-Patent Document 6: Anke M Tukker et al., Toxicological Sciences, Vol. 178, pp. 71-87, 2020
Non-Patent Document 7: Leonid Tarassishin et al., Glia, Vol. 62, pp. 999-1013, 2014

### Summary of Invention

### Object to be solved by the invention

An object to be achieved by the present invention is to provide a production method of a three-dimensional cell culture containing human-derived neurons, human-derived astrocytes, and human-derived microglia capable of mimicking human brain functions, and a culture method of a three-dimensional cell culture containing human-derived neurons, human-derived astrocytes, and human-derived microglia capable of mimicking human brain functions. Another object to be achieved by the present invention is to provide a three-dimensional cell culture containing human-derived neurons, human-derived astrocytes, and human-derived microglia capable of mimicking human brain functions. Another object to be achieved by the present invention is to provide an evaluation method of a test substance using the above-described three-dimensional cell culture.

### Means for solving the object

As a result of intensive studies to achieve the above object, the present inventors have found that a three-dimensional cell culture capable of mimicking human brain functions can be produced by adding human-derived astrocytes, human-derived neurons, and human-derived microglia to a culture vessel such that a proportion of the astrocytes among all cells added to the culture vessel is 30% or more prior to co-culture of the cells. The present invention has been completed based on these findings.

That is, according to an aspect of the present invention, the following invention is provided.
<1> A production method of a three-dimensional cell culture, the production method comprising a step of adding, prior to co-culture of cells, human-derived astrocytes, human-derived neurons, and human-derived microglia to a culture vessel, and a step of co-culturing the astrocytes, the neurons, and the microglia in the culture vessel, in which a proportion of the astrocytes among all cells added to the culture vessel is 30% or more.
<2> The production method according to <1>, in which the astrocytes, the neurons, and the microglia are simultaneously added to the culture vessel.
<3> The production method according to <1> or <2>, in which the step of adding, prior to co-culture of cells, human-derived astrocytes, human-derived neurons, and human-derived microglia to the culture vessel includes a step of suspending the human-derived astrocytes, the human-derived neurons, and the human-derived microglia in a culture medium, and a step of simultaneously adding the culture medium containing the astrocytes, the neurons, and the microglia obtained by the step to the culture vessel.
<4> The production method according to any one of <1> to <3>, in which the astrocytes, the neurons, and the microglia are induced to differentiate from human-derived pluripotent stem cells.
<5> The production method according to <4>, in which human-derived pluripotent stem cells are human iPS cells.
<6> The production method according to any one of <1> to <5>, in which a proportion of the microglia among all cells added to the culture vessel is 50% or less.
<7> The production method according to any one of <1> to <6>, in which a proportion of the neurons among all cells added to the culture vessel is 10% or more.
<8> The production method according to any one of <1> to <7>, in which the three-dimensional cell culture has a spheroid shape.
<9> A culture method of a three-dimensional cell culture, the culture method comprising a step of adding, prior to co-culture of cells, human-derived astrocytes, human-derived neurons, and human-derived microglia to a culture vessel, and a step of co-culturing the astrocytes, the neurons, and the microglia in the culture vessel, in which a proportion of the astrocytes among all cells added to the culture vessel is 30% or more.
<10> The culture method according to <9>, in which the astrocytes, the neurons, and the microglia are simultaneously added to the culture vessel.
<11> The culture method according to <9> or <10>, in which the step of adding, prior to co-culture of cells, human-derived astrocytes, human-derived neurons, and human-derived microglia to the culture vessel includes a step of suspending the human-derived astrocytes, the human-derived neurons, and the human-derived microglia in a culture medium, and a step of simultaneously adding the culture medium containing the astrocytes, the neurons, and the microglia obtained by the step to the culture vessel.
<12> The culture method according to any one of <9> to <11>, in which the astrocytes, the neurons, and the microglia are induced to differentiate from human-derived pluripotent stem cells.
<13> The culture method according to <12>, in which human-derived pluripotent stem cells are human iPS cells.
<14> The culture method according to any one of <9> to <13>, in which a proportion of the microglia among all cells added to the culture vessel is 50% or less.
<15> The culture method according to any one of <9> to <14>, in which a proportion of the neurons among all cells added to the culture vessel is 10% or more.
<16> The culture method according to any one of <9> to <15>, in which the three-dimensional cell culture has a spheroid shape.
<17> A three-dimensional cell culture comprising human-derived astrocytes, human-derived neurons, and human-derived microglia, in which the three-dimensional cell culture is obtained by the production method according to any one of <1> to <8>.
<18> An evaluation method of a test substance, comprising bringing the three-dimensional cell culture according to <17> into contact with a test substance.
<19> An evaluation method of a test substance, comprising producing a three-dimensional cell culture by the production method according to any one of <1> to <8>, and bringing the three-dimensional cell culture into contact with a test substance.

### Effect of the invention

The three-dimensional cell culture produced by the method according to the embodiment of the present invention is a culture system mimicking a human brain, and is useful for research on intercellular interactions between neurons and glial cells in normal brain functions, or for research on pathological mechanisms.

### BRIEF DESCRIPTION OF THE DRAWINGS

[Fig.1] FIG. 1 shows the results of immunostaining of a three-dimensional co-culture.
[Fig.2] FIG. 2 shows the results of calcium imaging in the three-dimensional co-culture.

### Embodiments for carrying out the invention

Hereinafter, embodiments of the present invention will be specifically described.

The production method of a three-dimensional cell culture according to the embodiment of the present invention and the culture method of a three-dimensional cell culture according to the embodiment of the present invention are methods including: a step of adding, prior to co-culture of cells, human-derived astrocytes, human-derived neurons, and human-derived microglia to a culture vessel, and a step of co-culturing the astrocytes, the neurons, and the microglia in the culture vessel, in which a proportion of the astrocytes among all cells added to the culture vessel is 30% or more.

The "prior to co-culture of cells" means that the co-culture is not considered to be started within, for example, 1 to 24 hours after the addition of one or two types of cells.

A proportion of the astrocytes among all cells added to the culture container is substantially the same as a proportion of the astrocytes in the three-dimensional cell culture. A proportion of the neurons among all cells added to the culture container is substantially the same as a proportion of the neurons in the three-dimensional cell culture. A proportion of the microglia among all cells added to the culture container is substantially the same as a proportion of the microglia in the three-dimensional cell culture.

In the present invention, it is preferable that the astrocytes, the neurons, and the microglia can be simultaneously added to the culture vessel.

Specifically, for example, the three types of cells may be added to the culture vessel after being suspended and mixed in a culture medium in one container. In this case, the step of adding human-derived astrocytes, human-derived neurons, and human-derived microglia to a culture vessel includes a step of suspending the human-derived astrocytes, human-derived neurons, and human-derived microglia in a culture medium, and a step of simultaneously adding the culture medium containing the human-derived astrocytes, human-derived neurons, and human-derived microglia obtained as described above to the culture vessel.

Alternatively, the three types of cells described above may be separately suspended in separate culture media and may be simultaneously added to the same culture vessel. Alternatively, three types of frozen cells frozen and stored separately may be thawed and simultaneously added to the same culture vessel.

However, the aspect in which the astrocytes, the neurons, and the microglia are simultaneously added to the culture vessel is not limited to the above.

In the present invention, three types of cells (astrocytes, neurons, and microglia) can be co-cultured at any proportion by simultaneously being seeded. In addition, by three-dimensional co-culturing three types of cells (astrocytes, neurons, and microglia), it is possible to reflect and mimic the human brain more than the conventional culture system, which is useful in studying the original function of the brain and the mechanism of disease formation. In the present invention, since three types of cells are used, it is possible to evaluate the intercellular interactions in brain function or pathogenesis.

By three-dimensional culturing the three types of cells, the neural activity evaluation can be performed after 3 weeks of culture. Since the neural activity evaluation in the two-dimensional co-culture of the three types of cells requires 4 weeks of culture, the three-dimensional culture may promote the construction of the network of the neurons.

In addition, by simultaneously seeding the three types of cells, the influence of the glial cells in the process of the neurons constructing a network or the process of the neurons maturing can be evaluated.

The astrocytes, the neurons, and the microglia are preferably induced to differentiate from human-derived pluripotent stem cells.

Examples of the human-derived pluripotent stem cells include human induced pluripotent stem cells (human iPS cells), human embryonic stem cells (human ES cells), human mesenchymal stem cells, and the like. Human iPS cells are preferable, but the human-derived pluripotent stem cell is not particularly limited thereto. The human iPS cell is an iPS cell produced from a human cell.

Examples of the human-derived pluripotent stem cells include pluripotent stem cells derived from a sample in which there is no mutation in a disease-related gene that causes a neurological disease, pluripotent stem cells derived from a sample collected from a healthy human (healthy person) who does not have a neurological disease, pluripotent stem cells derived from a sample collected from a patient having a disease, and pluripotent stem cells derived from a sample in which there is a mutation in a disease-related gene. The "there is no mutation in a disease-related gene" means that there is no mutation that causes a nervous system disease in the disease-related gene. That is, in a case where there is a mutation in a gene but the mutation does not cause a disease, it is interpreted that there is no mutation in the disease-related gene. In addition, in a case of "there is a mutation in a disease-related gene", the mutation may be an endogenous genetic mutation originally possessed by a patient, or an exogenous genetic mutation introduced artificially. It is presumed that, by producing the three-dimensional cell culture according to the embodiment of the present invention using the pluripotent stem cells derived from a sample collected from a patient having a disease or the pluripotent stem cells derived from a sample in which there is a mutation in a disease-related gene, the pathological conditions of a disease derived from a sample or a disease caused by a mutation in a disease-related gene can be mimicked.

The ES cells can be established, for example, by culturing an early embryo before implantation, an inner cell mass constituting the early embryo, a single-cell embryo, or the like (Manipulating the Mouse Embryo, A Laboratory Manual, Second Edition, Cold Spring Harbor Laboratory Press, 1994, Thompson, J. A. et al.: Science, 282, 1145-1147, 1998). As the early embryo, an early embryo produced by nuclear transplantation of a somatic cell nucleus may be used (Wilmut et al.: Nature, 385, 810, 1997; Cibelli et al.: Science, 280, 1256, 1998; Akira Iritani et al.: Protein, Nucleic Acid and Enzyme, 44, 892, 1999; Baguisi et al.: Nature Biotechnology, 17, 456, 1999; Wakayama et al.: Nature, 394, 369, 1998, Nature Genetics, 22, 127, 1999, Proc. Natl. Acad. Sci. USA, 96, 14984, 1999; Rideout III et al.: Nature Genetics, 24, 109, 2000; Tachibana et al.: Human Embryonic Stem Cells Derived by Somatic Cell Nuclear Transfer, Cell, 2013, in press). As the early embryo, a parthenogenetic embryo may be used (Kim et al.: Science, 315, 482-486, 2007; Nakajima et al.: Stem Cells, 25, 983-985, 2007; Kim et al.: Cell Stem Cell, 1, 346-352, 2007; Revazova et al.: Cloning Stem Cells, 9, 432-449, 2007; Revazova et al.: Cloning Stem Cells, 10, 11-24, 2008). In addition to the above-described papers, for the production of the ES cells, Strelchenko N, et al.: Reprod Biomed Online. 9:623-629, 2004, Klimanskaya I, et al.: Nature 444:481-485, 2006, Chung Y, et al.: Cell Stem Cell 2:113-117, 2008, Zhang X, et al.: Stem Cells 24:2669-2676, 2006, Wassarman, P. M. et al.: Methods in Enzymology, Vol. 365, 2003, and the like are useful as references. It is noted that a fused ES cell obtained by cell fusion of an ES cell with a somatic cell is also included in the embryonic stem cell that is used in the method according to the embodiment of the present invention.

Some ES cells are available from conservation institutions or are commercially available. For example, human ES cells are available from the Institute for Frontier Medical Sciences, Kyoto University (for example, KhES-1, KhES-2, and KhES-3), WiCell Research Institute, ESI BIO.

The iPS cell means a cell that is produced by reprogramming a somatic cell by introducing reprogramming factors and has pluripotency (multiple differentiation potency) and proliferation ability. The iPS cells exhibit properties similar to the ES cells. The somatic cells used for producing iPS cells are not particularly limited and may be differentiated somatic cells or undifferentiated stem cells. The iPS cell can be prepared by a known method or the like. In addition, it is naturally expected that an iPS cell production method to be developed in the future will be applied.

The most basic method for producing the iPS cells is a method of introducing four factors of Oct3/4, Sox2, Klf4, and c-Myc, which are transcription factors, into cells using a virus (Takahashi K, Yamanaka S: Cell 126 (4), 663-676, 2006; Takahashi K, et al.: Cell 131 (5), 861-72, 2007). For the human iPS cells, there is a report of establishment by introduction of four factors of Oct4, Sox2, Lin28, and Nanog (Yu J, et al.: Science 318 (5858), 1917-1920, 2007). The establishment of iPS cells by introduction of three factors excluding c-Myc (Nakagawa M, et al.: Nat. Biotechnol. 26 (1), 101-106, 2008), two factors of Oct3/4 and Klf4 (Kim J B, et al.: Nature 454 (7204), 646-650, 2008), or only Oct3/4 (Kim J B, et al.: Cell 136 (3), 411-419, 2009) has also been reported. In addition, a method of introducing a protein, which is an expression product of a gene, into a cell has also been reported (Zhou H, Wu S, Joo JY, et al.: Cell Stem Cell 4, 381-384, 2009; Kim D, Kim CH, Moon JI, et al.: Cell Stem Cell 4, 472-476, 2009). On the other hand, it has also been reported that the production efficiency can be improved or the number of factors to be introduced can be reduced by using an inhibitor BIX-01294 for histone methyltransferase G9a, a histone deacetylase inhibitor valproic acid (VPA), BayK8644, or the like (Huangfu D, et al.: Nat. Biotechnol. 26 (7), 795-797, 2008; Huangfu D, et al.: Nat. Biotechnol. 26 (11), 1269-1275, 2008; Silva J, et al.: PLoS. Biol. 6 (10), e253, 2008). Studies on the gene introduction method are also being advanced, and in addition to the retrovirus, a technology has been developed in which a lentivirus (Yu J, et al.: Science 318 (5858), 1917-1920, 2007), an adenovirus (Stadtfeld M, et al.: Science 322 (5903), 945-949, 2008), a plasmid (Okita K, et al.: Science 322 (5903), 949-953, 2008), a transposon vector (Woltjen K, Michael IP, Mohseni P, et al.: Nature 458, 766-770, 2009; Kaji K, Norrby K, Pac a A, et al.: Nature 458, 771-775, 2009; Yusa K, Rad R, Takeda J, et al.: Nat Methods 6, 363-369, 2009), or an episomal vector (Yu J, Hu K, Smuga-Otto K, Tian S, et al.: Science 324, 797-801, 2009) is used for gene introduction.

Cells transformed to iPS cells, that is, cells that have undergone initialization (reprogramming) can be selected using, as an index, the expression of pluripotent stem cell markers (undifferentiated markers) such as Fbxo15, Nanog, Oct3/4, Fgf-4, Esg-1, and Cript, or the like. The selected cells can be recovered as iPS cells.

As a method of producing the iPS cells, in addition to a method of manufacturing the iPS cells by direct initialization using gene expression, the iPS cells can also be induced from the somatic cells by adding a compound (Hou P, et al.: Science 341 (6146), 651-654, 2013).

The iPS cells can be provided from, for example, FUJIFILM Cellular Dynamics, Inc. (FCDI), National University Corporation Kyoto University, or Independent Administrative Institution RIKEN BioResource Center.

Examples of the human-derived astrocytes include astrocytes induced to differentiate from astrocyte precursor cells (XCell Science, XCS-AP-001-1V, and the like), iCell (registered trademark) astrocytes (FUJIFILM Cellular Dynamics, C1037), and the like, and astrocytes induced to differentiate from astrocyte precursor cells are preferable. In a case of inducing the astrocyte precursor cells to differentiate into the astrocytes, it is preferable to induce the astrocyte precursor cells to differentiate in the absence of serum.

Examples of the method for obtaining human-derived neurons include inducing from human iPS cells produced from somatic cells collected from healthy humans (healthy person) who do not have a mutation of a disease-related gene causing a nervous system disease or who do not have a nervous system disease, or from somatic cells collected from patients who have a nervous system disease, and inducing from an established human iPS cell.

The neurons induced to differentiate from the human-derived pluripotent stem cells are not particularly limited, but are preferably motor neurons, cerebral cortex excitatory neurons, or substantia nigra neurons.

A method for inducing human-derived pluripotent stem cells to differentiate into neurons is not particularly limited, but includes a method of producing neural stem cells from pluripotent stem cells using a low-molecular-weight compound treatment or the like and then inducing to differentiate into neurons, and a method of directly inducing to differentiate into neurons by gene expression or the like.

Examples of the method of inducing the pluripotent stem cells to differentiate into the neurons include:
(1) a method of forming and differentiating embryoid bodies (cell aggregates containing neural precursor cells) by culturing in a serum-free medium (SFEB method: Watanabe K, et al., Nat. Neurosci., 8:288-296, 2005; SFEBq method: Wataya T, et al. Proc. Natl. Acad. Sci. USA., 105:11796-11801, 2008);
(2) a method of differentiating by culturing on stromal cells (SDIA method: Kawasaki H, et al., Neuron, 28:31-40, 2000);
(3) a method of differentiating by culturing on Matrigel to which a drug has been added (Chambers S. M, et al., Nat. Biotechnol., 27:275-280, 2009);
(4) a method of differentiating by culturing in a culture medium containing a low-molecular-weight compound as a substitute for a cytokine (US5843780A),
(5) a method of differentiating by introducing a neurogenic factor (a gene encoding Neurogenin2 (NGN2) protein or the like) into the pluripotent stem cells and expressing the factor (WO2014/148646A; and Zhang Y, et al., Neuron, 78:785-98, 2013); and
(6) a method of differentiating by introducing and expressing miR-9/9*-124 in pluripotent stem cells; and
a combination of these methods.

Among the above, (5) the method of introducing a gene encoding an NGN2 protein into the pluripotent stem cells and expressing the protein is preferable because matured neurons can be obtained in a short period of time and with high efficiency.

Examples of the human-derived neurons include induced neurons differentiated from iPS cells by forced expression of NGN2, iCell (registered trademark) Gluta Neurons (FUJIFILM Cellular Dynamics, C1033), iCell (registered trademark) GABA Neurons (FUJIFILM Cellular Dynamics, C1008), iCell (registered trademark) Dopa Neurons (FUJIFILM Cellular Dynamics, C1028), iCell (registered trademark) Motor Neurons (FUJIFILM Cellular Dynamics, C1048), and the like. Among these, the induced neurons differentiated from iPS cells by forced expression of NGN2 and the iCell (registered trademark) Gluta Neurons are preferable.

Examples of the human-derived microglia include iCell (registered trademark) Microglia (FUJIFILM Cellular Dynamics, C1110), microglia (Axol Bioscience, AX0664), and the like. Among these, the iCell (registered trademark) Microglia is preferable.

The astrocytes are cells that express at least one or more marker genes specific to the astrocytes consisting of glial fibrillary acidic protein (GFAP), S100 calcium binding protein B (S100β), potassium inwardly rectifying channel subfamily J member 10 (KCNJ10), Aquaporin-4 (AQP4), and solute carrier family 1 member 3 (SLC1A3).

The neurons are preferably cells which express at least one or more marker genes specific to neurons consisting of β-III tubulin, NeuN, a neural cell adhesion molecule (N-CAM), and microtubule-associated protein 2 (MAP2), and have a β-III tubulin-positive protrusion (hereinafter, referred to as a neurite).

Microglia are cells that express at least one or more marker genes specific to microglia consisting of ionized calcium-binding adapter molecule 1 (IBA1), CD33, CD45, triggering receptor expressed on myeloid cells 2 (TREM2), purinergic receptor P2Y (P2RY12, G-protein coupled 12), transmembrane protein 119 (TMEM119), and CX3C-chemokine receptor 1 (CX3CR1).

The expression level of a marker gene can be usually analyzed by the production amount of a transcript corresponding to the gene, or the production amount, the activity, and the like of a translation product thereof. The measurement of the expression level can be carried out by measuring an mRNA which is a transcript of a gene or a protein which is a translation product of a gene; however, it is preferably carried out by measuring an mRNA or a cDNA which is a reverse transcript thereof. The detection or measurement of the expression of the translation product (protein) can be performed by immunocytochemistry for detecting the protein in the cell using an antibody.

In the present invention, a proportion of the astrocytes among all cells added to the culture vessel is 30% or more, preferably 35% or more, more preferably 40% or more, and still more preferably 45% or more. The upper limit of the proportion of the astrocytes among all cells added to the culture vessel is not particularly limited, but is generally 80% or less and preferably 70% or less.

By setting the proportion of the astrocytes among all cells added to the culture vessel to 30% or more, the maturation of the neurons is promoted, and in the neural activity evaluation by calcium imaging or the like, calcium oscillation, which is an indicator of the maturation and network construction of the neurons, can be stably and clearly detected.

A proportion of the microglia among all cells added to the culture vessel is preferably 50% or less, more preferably 40% or less, and still more preferably 30% or less. The lower limit of the proportion of the microglia among all cells added to the culture vessel is not particularly limited, but is generally 0.1% or more, preferably 1% or more, and more preferably 10% or more.

By setting the proportion of the microglia to 0.1% or more, it is possible to confirm that the microglia activated by neuroinflammation affects other cells, and it is possible to evaluate the intercellular interaction during neuroinflammation.

A proportion of the neurons among all cells added to the culture vessel is preferably 10% or more, more preferably 15% or more, still more preferably 20% or more, and particularly preferably 25% or more. An upper limit of the proportion of the neurons is not particularly limited, but is generally 65% or less and preferably 60% or less.

By setting the proportion of the neurons to 10% or more, calcium oscillation can be clearly detected by calcium imaging, which is useful for the neural activity evaluation. **In** addition, in the present invention, it is possible to confirm by immunostaining that the neurons, the astrocytes, and the microglia can be cultured for a long period of time, and the influence of the glial cells on the neural activity can be evaluated.

**In** the present invention, the astrocytes, the neurons, and the microglia are co-cultured in a culture vessel.

As the culture container, a plate having wells, a dish, a cell culture insert, a cell culture flask, or the like can be used, and the plate having wells is preferable. Specific examples thereof include PrimeSurface (registered trademark) plate (Sumitomo Bakelite) and cell-repellent plate (Greiner Bio-one), and a PrimeSurface plate (Sumitomo Bakelite) is preferable.

Examples of the shape of the culture vessel include a round bottom, a U-shaped bottom, a V-shaped bottom, and the like, but the shape is not particularly limited.

The properties of the surface with which the culture medium in the container comes into contact are preferably cell non-adhesiveness. As a result, the cells are cultured in a floating state, and a spheroid is easily formed. Alternatively, only a certain portion of the surface inside the container may be cell adhesiveness, and the other portions may be cell non-adhesiveness. **In** this case, cells can be gathered at the portion with cell adhesiveness to form a spheroid.

The lower limit value of the number of cells (the number of cells is the total number of cells of three types of cells, the same applies to the following) at the time of seeding into the 96-well plate is not particularly limited, but it is, for example, preferably 0.7 × 10⁴ cells/well or more, more preferably 1.0 × 10⁴ cells/well or more, still more preferably 1.2 × 10⁴ cells/well or more, even still more preferably 1.4 × 10⁴ cells/well or more, particularly preferably 1.6 × 10⁴ cells/well or more, and most preferably 1.8 × 10⁴ cells/well or more. The upper limit value of the cell density at the time of seeding in the culture container is not particularly limited, but for example, may be 8.0 × 10⁴ cells/well or less, and is preferably less than 8.0 × 10⁴ cells/well, more preferably 5.0 × 10⁴ cells/well or less, still more preferably 3.0 × 10⁴ cells/well or less, even still more preferably 2.5 × 10⁴ cells/well or less, and particularly preferably 2.3 × 10⁴ cells/well or less.

The number of cells at the time of seeding into the 96-well plate is preferably 1.0 × 10⁴ cells/well or more and 8.0 × 10⁴ cells/well or less, more preferably 1.2 × 10⁴ cells/well or more and 5.0 × 10⁴ cells/well or less, still more preferably 1.4 × 10⁴ cells/well or more and 3.0 × 10⁴ cells/well or less, even still more preferably 1.6 × 10⁴ cells/well or more and 2.5 × 10⁴ cells/well or less, and particularly preferably 1.8 × 10⁴ cells/well or more and 2.3 × 10⁴ cells/well or less.

A medium can be selected from the known media and commercially available media. The culture medium used for the culture can be used by adding an additive to the basal medium. Here, examples of the basal medium include DMEM, DMEM (Dulbecco's modified Eagle's medium)/F12, BrainPhys (registered trademark) Neuronal Medium, Neurobasal (trademark) Medium-A, Neurobasal (trademark) Medium, Neural Progenitor Basal Medium, NS-A Basal Medium, Basal Medium Eagle (BME), BGJb Medium, CMRL 1066 Medium, Glasgow Minimum Essential Medium (MEM), Improved MEM Zinc Option, Iscove's Modified Dulbecco's Medium (IMDM), Medium 199, Eagle MEM, αMEM, Ham's F12 Medium, RPMI 1640 Medium, Fischer's Medium, and the like. In addition, as the culture medium, a single culture medium may be used, or two or more culture media may be used in combination.

Specific examples of the additive that can be added to the culture medium include serum, retinoic acid, Wnt, bone morphogenetic protein (BMP) (BMP-4 and the like), ciliary neurotrophic factor (CNTF), brain-derived neurotrophic factor (BDNF), glial cell line-derived neurotrophic factor (GDNF), basic fibroblast growth factor (bFGF), epidermal growth factor (EGF), hepatocyte growth factor (HGF), sonic hedgehog (SHH), insulin-like growth factor 1 (IGF-1), Activin A, Heregulinβ-1, interleukins, 8-Br-cAMP, heparin, heparan sulfate, laminin, collagen, fibronectin, progesterone, selenite, B-27 (registered trademark) supplement, N2 supplement with transferrin (Apo), N2 supplement with transferrin, GlutaMAX (trademark), L(+)-ascorbic acid, ITS supplement, MEM non-essential amino acids, GlutaMAX (trademark) supplement, and the like, but the additive is not particularly limited. Furthermore, an antibiotic (penicillin, streptomycin, or the like) may be added.

As the culture conditions, general cell culture conditions may be selected. For example, conditions of 37°C and 5% CO₂.are mentioned. The culture medium may be exchanged at appropriate intervals (preferably once every 1 to 7 days and more preferably once every 2 to 3 days) during the culture, but the culture medium may not be exchanged during the culture period.

The three-dimensional cell culture produced in the present invention preferably has a spheroid shape. The spheroid can be formed by co-culturing the astrocytes, the neurons, and the microglia in the culture vessel. Alternatively, the three-dimensional cell culture can also be produced by co-culturing the astrocytes, the neurons, and the microglia in a culture solution in which the culture solution is gelled in the culture vessel, and gelling the culture solution after the co-culture.

According to the present invention, a three-dimensional cell culture containing the human-derived astrocytes, the human-derived neurons, and the human-derived microglia, which are obtained by the production method of a three-dimensional cell culture according to the embodiment of the present invention described above, is provided. **In** the present invention, since the human-derived astrocytes, the human-derived neurons, and the human-derived microglia are added to the culture vessel prior to co-culture of the cells, it is presumed that in the three-dimensional cell culture according to the embodiment of the present invention, the astrocytes, the neurons, and the microglia are present and distributed throughout the core and the surface of the three-dimensional cell culture.

Since the three-dimensional cell culture according to the embodiment of the present invention is obtained by culturing the three types of cells (astrocytes, neurons, and microglia) in three dimensions, the human brain can be reflected and mimicked, and it can be used for, for example, screening of new drugs useful for neurodegenerative diseases. Examples of the neurodegenerative disease include Alzheimer's disease (AD), spinocerebellar degeneration, frontotemporal lobar degeneration (FTLD), Parkinson's disease, amyotrophic lateral sclerosis (ALS), dementia with Lewy bodies, Huntington's disease, Niemann-Pick disease, and the like.

That is, according to the present invention, an evaluation method of a test substance, including bringing the three-dimensional cell culture according to the embodiment of the present invention into contact with a test substance, is provided. According to the present invention, furthermroe, an evaluation method of a test substance, including producing a three-dimensional cell culture by the production method according to the embodiment of the present invention, and bringing the three-dimensional cell culture into contact with a test substance.

More specifically, according to the present invention, there is further provided a method for screening a drug, including:
(1) a step of bringing the three-dimensional cell culture according to the embodiment of the present invention into contact with a test substance;
(2) a step of culturing the three-dimensional cell culture according to the embodiment of the present invention, which has been brought into contact with the test substance in the step (1), and the three-dimensional cell culture according to the embodiment of the present invention, which has not been brought into contact with the test substance, as a control;
(3) a step of measuring a function of the three-dimensional cell culture (for example, glutamine uptake, calcium oscillation, and the like); and
(4) a step of selecting a test substance that changes the function, as a candidate drug, in comparison with a control that has not been brought into contact with the test substance.

Examples of the test substance include a protein, a peptide, an antibody, a nucleic acid (a gene expression vector, siRNA, miRNA, an antisense oligonucleotide, mRNA, or the like), a virus vector (AAV, lentivirus, adenovirus, or the like), a non-peptidic compound, a synthetic compound, a synthetic low-molecular-weight compound, a natural compound, a cell extract, an extracellular vesicle, a plant extract, an animal tissue extract, a blood plasma, an extract derived from a marine organism, a cell culture supernatant, a microbial fermentation product, and the like.

In addition, the test substance can be obtained by using any of a plurality of approaches in a combinatorial library method known in the related art, including (1) biological library method, (2) synthetic library method using deconvolution, (3) one-bead one-compound library method, and (4) synthetic library method using affinity chromatography sorting. The biological library method using affinity chromatography sorting is limited to a peptide library, but other approaches can be applied to a low-molecular-weight compound library of peptides, non-peptide oligomers, or compounds (Lam (1997) Anticancer Drug Des. 12: 145-67). Examples of the synthesis method of the molecular library can be found in the related art (DeWitt et al. (1993) Proc. Natl. Acad. Sci. USA 90:6909-13; Erb et al. (1994) Proc. Natl. Acad. Sci. USA 91:11422-6; Zuckermann et al. (1994) J. Med. Chem. 37:2678-85; Cho et al. (1993) Science 261:1303-5; Carell et al. (1994) Angew. Chem. Int. Ed. Engl. 33:2059; Carell et.al. (1994) Angew. Chem. Int. Ed. Engl. 33:2061; Gallop et al. (1994) J. Med. Chem. 37:1233-51). The compound library can be produced as a solution (see Houghten (1992) Bio/Techniques 13:412-21), beads (Lam (1991) Nature 354:82-4), tips (Fodor (1993) Nature 364:555-6), bacteria (US5223409A), spores (US5571698A, US5403484A, and US5223409A), plasmids (Cull et al. (1992) Proc. Natl. Acad. Sci. USA 89:1865-9), or phages (Scott and Smith (1990) Science 249:386-90; Devlin (1990) Science 249:404-6; Cwirla et al. (1990) Proc. Natl. Acad. Sci. USA 87:6378-82; Felici (1991) J. Mol. Biol. 222:301-10; US2002/0103360A).

Bringing the three-dimensional cell culture into contact with the test substance may be performed by adding the test substance to a culture solution of the three-dimensional cell culture. The contact is not particularly limited as long as the change in the index can be confirmed, but is, for example, 1 day or more, 2 days or more, 3 days or more, 4 days or more, 5 days or more, 6 days or more, or 7 days or more. The concentration of the test substance to be added can be appropriately adjusted depending on the properties of the compound (solubility, toxicity, and the like).

The culture solution of three-dimensional cell culture, which is used in a case of bringing the three-dimensional cell culture into contact with a test substance, is not particularly limited as long as it is a culture medium in which three-dimensional cell culture can be cultured.

The culture temperature in a case where the three-dimensional cell culture is brought into contact with the test substance is not particularly limited, but is approximately 30°C to 40°C and preferably about 37°C, and the culture is performed in an atmosphere of CO₂-containing air, and the CO₂ concentration is preferably about 2% to 5%.

The present invention will be more specifically described with reference to Examples, but the present invention is not limited to the scope of Examples.

### Examples

### <Production of three-dimensional co-culture>

Human iPS cell-derived astrocyte progenitor cells (XCell Science, XCS-AP-001-1V) were seeded in a flask coated with Matrigel basement membrane matrix (Corning, 354234) and cultured for 5 days (37°C, 5% CO₂) using a differentiation-inducing culture medium to differentiation-induce into astrocytes.

**[Table 1]**

| Differentiation-inducing culture medium | | |
|---|---|---|
| Reagent name | Manufacturer/Model number | Final concentration |
| N2 Supplement with Transferrin (Apo) (×100) | FUJIFILM Wako Pure Chemical Corporation, 141-09041 | ×1 |
| GlutaMAX (trademark) | Thermo Fisher Scientific, 35050-061 | 1% |
| MEM Non-Essential Amino Acid | Thermo Fisher Scientific, 11140-050 | 1% |
| Penicillin-Streptomycin Solution (×100) | FUJIFILM Wako Pure Chemical Corporation, 168-23191 | ×0.5 |
| Heparin Sodium | FUJIFILM Wako Pure Chemical Corporation, 085-00134 | 0.5 U/mL |
| Animal-Free Recombinant Human CNTF | PeproTech, AF-450-13 | 10 ng/mL |
| Animal-Free Recombinant Human BMP-4 | PeproTech, AF-120-05ET | 10 ng/mL |

The differentiated astrocytes were peeled by TrypLE (trademark) Select (Thermo Fisher Scientific, 12563-029) and centrifuged at 600 × g at room temperature for 5 minutes. After centrifugation, the supernatant was removed, the cells were suspended in 1 mL of the co-culture medium, and the number of cells was counted.

The neurons produced by forcing expression of the Ngn2 gene from iPS cells (WO2014/148646A; and Zhang Y, et al., Neuron, 78:785-98, 2013) were thawed in a warm bath at 37°C. After thawing, the cells were added to the co-culture medium and centrifuged at 600 × g at room temperature for 5 minutes. After centrifugation, the supernatant was removed, the cells were suspended in 1 mL of the co-culture medium, and the number of cells was counted.

The iPS cell-derived microglia (iCell (registered trademark) Microglia, FCDI, C1110) were thawed in a warm bath at 37°C. After thawing, the cells were added to the co-culture medium and centrifuged at 600 × g at room temperature for 5 minutes. After centrifugation, the supernatant was removed, the cells were suspended in 1 mL of the co-culture medium, and the number of cells was counted.

The astrocytes, the neurons, and the microglia were mixed at a ratio of 10:6:3 (53%:32%:16%), and seeded into a 96-well plate (PrimeSurface (registered trademark) plate 96U, Sumitomo Bakelite, MS-9096U) at 1.9 × 10⁴ cells/well. The cells were cultured under the conditions of 37°C and 5% CO₂, and half of the culture medium was replaced three times a week.

**[Table 2]**

| Co-culture medium | | |
|---|---|---|
| Reagent name | Manufacturer/Model number | Final concentration |
| N2 Supplement with Transferrin (Apo) (×100) | FUJIFILM Wako Pure Chemical Corporation, 141-09041 | ×1 |
| GlutaMAX (trademark) | Thermo Fisher Scientific, 35050-061 | 1% |
| MEM Non-Essential Amino Acid | Thermo Fisher Scientific, 11140-050 | 1% |
| Penicillin-Streptomycin Solution (×100) | FUJIFILM Wako Pure Chemical Corporation, 168-23191 | ×0.5 |
| Animal-Free Recombinant Human CNTF | PeproTech, AF-450-13 | 10 ng/mL |
| Animal-Free Recombinant Human BMP-4 | PeproTech, AF-120-05ET | 10 ng/mL |
| 8-Br-cAMP | Sigma Aldrich, B7880 | 1 µmol/L |
| Animal-Free Recombinant Human/Murine/Rat BDNF | Peprotech, AF-450-02 | 20 ng/mL |
| Recombinant Human GDNF | Peprotech, 450-10 | 20 ng/mL |
| L(+)-Ascorbic Acid | FUJIFILM Wako Pure Chemical Corporation, 012-04802 | 20 nmol/L |
| Laminin | Sigma Aldrich, L2020 | 1 µg/mL |
| B-27 (registered trademark) Supplement (50X), serum free | Thermo Fisher Scientific, 17504044 | ×0.5 |

### <Cell fixation and immunostaining>

The three-dimensional co-culture product that had been cultured for 3 weeks was fixed by treating with 4% paraformaldehyde-phosphate buffer solution (FUJIFILM Wako Pure Chemical Corporation, 161-20141) for 30 minutes. After washing with PBS (-), the co-culture was treated with Triton (registered trademark) X-100 solution (BioVision, 2104-100) diluted to 0.4% with PBS (-) for 15 minutes to perform permeation treatment. After the permeation treatment, the co-culture was treated with a BSA solution (Sigma-Aldrich, A416) adjusted at a concentration of 4% with PBS(-) for 30 minutes to perform blocking. Then, as a primary antibody reaction, an anti-MAP2 antibody (Novus, NB300-213) diluted 600-fold, an anti-IBA1 antibody (FUJIFILM Wako Pure Chemical Corporation, 019-19741) diluted 600-fold, and an anti-GFAP antibody (Merck, MAB3402) diluted 3000-fold were treated and allowed to stand overnight at 4°C.

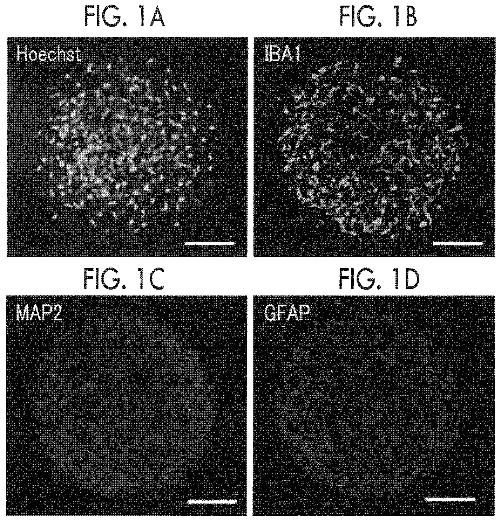

The next day, after washing with PBS (-), as a secondary antibody reaction, Goat anti-rabbit Alexa Fluor (registered trademark) 488 (Thermo Fisher Scientific, Inc., A11008) diluted 1,000 times, Goat anti-Chicken Alexa Fluor (registered trademark) 594 (Thermo Fisher Scientific, Inc., A11042) diluted 1,000 times, Goat anti-Mouse Alexa Fluor (registered trademark) 647 (Thermo Fisher Scientific, Inc., A32728) diluted 1,000 times, and Hoechst (registered trademark) 33342 solution (Dojindo, H342) diluted 1,000 times were treated and allowed to stand at room temperature for 60 minutes. After washing with PBS(-), imaging was performed with a confocal quantitative image cytometer CQ1 (Yokogawa Electric Corporation, Cell Voyager (registered trademark) CQ1) to acquire images. FIG. 1 shows the acquired image. (A) indicates nuclei (Hoechst), (B) indicates microglia (IBA1), (C) indicates neurons (MAP2), and (D) indicates astrocytes (GFAP), all of which were stained, and it was possible to detect signals in any of the cells. In addition, it was possible to confirm that the astrocytes, the neurons, and the microglia were present and distributed throughout the core and the surface of the three-dimensional co-culture.

### <Neural activity evaluation>

A working solution and a recording solution were prepared according to the following.

**[Table 3]**

| Working solution | | |
|---|---|---|
| Reagent name | Manufacturer/Model number | Final concentration |
| HBSS(-) without Phenol Red | FUJIFILM Wako Pure Chemical Corporation, 085-09355 | × 1 |
| Calcium Chloride Dihydrate | FUJIFILM Wako Pure Chemical Corporation, 036-19731 | 2.5 mmol/L |
| Calbrvte (trademark) 520AM | AAT Bioquest, 20651 | 4 µmol/L |
| Pluronic F-127 (20% solution in DMSO) | Thermo fisher scientific, P3000MP | 0.04% |
| HEPES buffer Solution | Dojindo, GB60 | 10 mmol/L |
| Hoechst (registered trademark) 33342 solution | Dojindo, H342 | × 1,000 |

**[Table 4]**

| Recording solution | | |
|---|---|---|
| Reagent name | Manufacturer/Model number | Final concentration |
| HBSS(-) without Phenol Red | FUJIFILM Wako Pure Chemical Corporation, 085-09355 | × 1 |
| Calcium Chloride Dihydrate | FUJIFILM Wako Pure Chemical Corporation, 036-19731 | 2.5 mmol/L |
| HEPES buffer Solution | Dojindo, GB60 | 10 mmol/L |

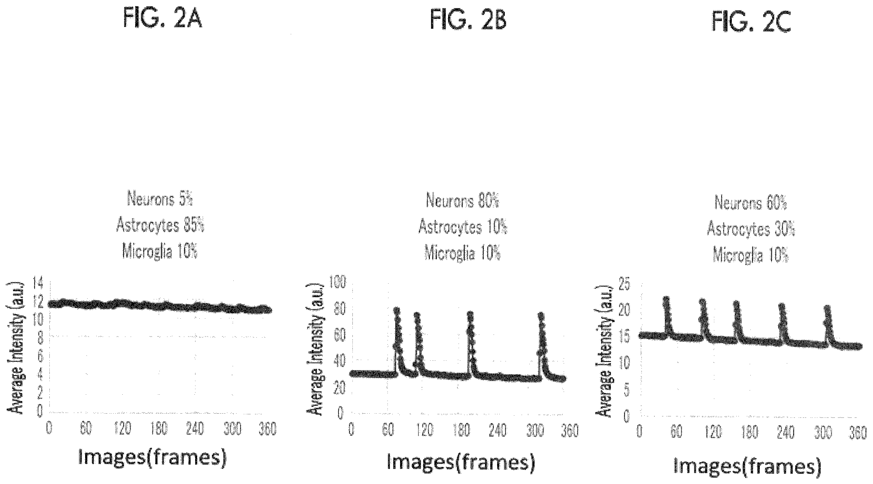

The three-dimensional co-culture produced by setting the proportion of the neurons, the astrocytes, and the microglia to 5:85:10, 80:10:10, or 60:30:10 was cultured for 3 weeks, an equal amount of the working solution to the culture medium was then added thereto, and the mixture was incubated at 37°C for 1 hour. Thereafter, the cells were washed with PBS(-), and an equal amount of the recording solution to PBS(-) was added thereto. Thereafter, a green fluorescent image of Calbryte (registered trademark) 520 AM was acquired with the confocal quantitative image cytometer CQ1 (3 images/sec, 2 minutes). Thereafter, the fluorescence intensity was quantified over time with ImageJ (NIH, version. 1.50i). The results are shown in FIG. 2. In the three-dimensional co-culture in which the proportion of the neurons was 5% (FIG. 2A), the intensity of the calcium oscillation (repeated influx of calcium ions in synchronization), which is an indicator of the neural activity, was weak and unclear. On the other hand, in the three-dimensional co-culture in which the proportion of the neurons was 80% and the proportion of the astrocytes was 10% (FIG. 2B), clear calcium oscillation was confirmed, but the interval was irregular. In the three-dimensional co-culture in which the proportion of the neurons was 60% and the proportion of the astrocytes was 30% (FIG. 2C), regular and clear calcium oscillation was confirmed. From this, it was found that the three-dimensional co-culture in which the proportion of the neurons was 10% or more and the proportion of the astrocytes was 30% or more was suitable for evaluating the intensity and frequency of the calcium oscillation.

### <Intercellular interactions during neuroinflammation>

The three-dimensional co-culture that had been cultured for 3 weeks was treated with Lipopolysaccharide (LPS) from E. coli 0127 (FUJIFILM Wako Pure Chemical Corporation, 124-05151) such that the final concentration was 100 ng/mL, and fixed by treating, for 30 minutes, with a 4% paraformaldehyde-phosphate buffer solution (FUJIFILM Wako Pure Chemical Corporation, 161-20141) at 0, 0.5, 1, 3, 6, and 24 hours after the treatment. After washing with PBS (-), the co-culture was treated with Triton (registered trademark) X-100 solution (BioVision, 2104-100) diluted to 0.4% with PBS (-) for 15 minutes to perform permeation treatment. After the permeation treatment, the co-culture was treated with a BSA solution (Sigma-Aldrich, A416) adjusted at a concentration of 4% with PBS(-) for 30 minutes to perform blocking. Thereafter, as a primary antibody reaction, the cells were treated with an anti-IBA1 antibody (FUJIFILM Wako Pure Chemical Corporation, 011-27991) diluted 1000-fold and an anti-NF-κB p65 antibody (Cell Signaling, 8242) diluted 800-fold, and allowed to stand at 4°C overnight.

The next day, the cells were washed with PBS(-), then as a secondary antibody reaction, treated with Donkey anti-goat Alexa Fluor (registered trademark) 488 (Thermo Fisher Scientific, A11055) diluted 1000-fold, Donkey anti-rabbit Alexa Fluor (registered trademark) 594 (Thermo Fisher Scientific, A32754) diluted 1000-fold, and Hoechst (registered trademark) 33342 solution (Dojindo, H342) diluted 1000-fold as a secondary antibody reaction, and allowed to stand at room temperature for 60 minutes. After washing with PB S(-), imaging was performed with a confocal quantitative image cytometer CQ1 (Yokogawa Electric Corporation, Cell Voyager (registered trademark) CQ1) to acquire images.

By observing the change in nuclear localization of NF-κB after the LPS treatment, it was possible to confirm that the activation of the microglia affects other cells, that is, intercellular interaction occurs.

## Claims

1. A production method of a three-dimensional cell culture, the production method comprising:
a step of adding, prior to co-culture of cells, human-derived astrocytes, human-derived neurons, and human-derived microglia to a culture vessel; and
a step of co-culturing the astrocytes, the neurons, and the microglia in the culture vessel,
wherein a proportion of the astrocytes among all cells added to the culture vessel is 30% or more.

2. The production method according to claim 1,
wherein the astrocytes, the neurons, and the microglia are simultaneously added to the culture vessel.

3. The production method according to claim 1,
wherein the step of adding, prior to co-culture of cells, human-derived astrocytes, human-derived neurons, and human-derived microglia to the culture vessel includes a step of suspending the human-derived astrocytes, the human-derived neurons, and the human-derived microglia in a culture medium, and a step of simultaneously adding the culture medium containing the astrocytes, the neurons, and the microglia obtained by the step to the culture vessel.

4. The production method according to claim 1,
wherein the astrocytes, the neurons, and the microglia are induced to differentiate from human-derived pluripotent stem cells.

5. The production method according to claim 4,
wherein the human-derived pluripotent stem cells are human iPS cells.

6. The production method according to any one of claims 1 to 5,
wherein a proportion of the microglia among all cells added to the culture vessel is 50% or less.

7. The production method according to any one of claims 1 to 5,
wherein a proportion of the neurons among all cells added to the culture vessel is 10% or more.

8. The production method according to any one of claims 1 to 5,
wherein the three-dimensional cell culture has a spheroid shape.

9. A culture method of a three-dimensional cell culture, the culture method comprising:
a step of adding, prior to co-culture of cells, human-derived astrocytes, human-derived neurons, and human-derived microglia to a culture vessel; and
a step of co-culturing the astrocytes, the neurons, and the microglia in the culture vessel,
wherein a proportion of the astrocytes among all cells added to the culture vessel is 30% or more.

10. The culture method according to claim 9,
wherein the astrocytes, the neurons, and the microglia are simultaneously added to the culture vessel.

11. The culture method according to claim 9,
wherein the step of adding, prior to co-culture of cells, human-derived astrocytes, human-derived neurons, and human-derived microglia to the culture vessel includes a step of suspending the human-derived astrocytes, the human-derived neurons, and the human-derived microglia in a culture medium, and a step of simultaneously adding the culture medium containing the astrocytes, the neurons, and the microglia obtained by the step to the culture vessel.

12. The culture method according to claim 9,
wherein the astrocytes, the neurons, and the microglia are induced to differentiate from human-derived pluripotent stem cells.

13. The culture method according to claim 12,
wherein the human-derived pluripotent stem cells are human iPS cells.

14. The culture method according to any one of claims 9 to 13,
wherein a proportion of the microglia among all cells added to the culture vessel is 50% or less.

15. The culture method according to any one of claims 9 to 13,
wherein a proportion of the neurons among all cells added to the culture vessel is 10% or more.

16. The culture method according to any one of claims 9 to 13,
wherein the three-dimensional cell culture has a spheroid shape.

17. A three-dimensional cell culture comprising:
human-derived astrocytes;
human-derived neurons; and
human-derived microglia,
wherein the three-dimensional cell culture is obtained by the production method according to any one of claims 1 to 5.

18. An evaluation method of a test substance, comprising:
bringing the three-dimensional cell culture according to claim 17 into contact with a test substance.

19. An evaluation method of a test substance, comprising:
producing a three-dimensional cell culture by the production method according to any one of claims 1 to 5; and
.bringing the three-dimensional cell culture into contact with a test substance.
